# EUROPEAN PATENT APPLICATION

(11) **EP 2 617 353 A1**
(43) Date of publication of application: **24.07.2013**
(21) Application number: 12152027.4
(22) Date of filing: 22.01.2012
(51) Int. Cl.: A61B 3/113, A61B 5/18, G02B 5/32

(54) **System for an observation of an eye and its surrounding area**

(71) Applicant: Université de Liège, 4031 Angleur (Liege) (BE)
(72) Inventor: Verly, Jacques, 4000 Liège (BE); Habraken, Serge, 4000 Liège 1 (BE)

(57) **Abstract**

System and device for an observation of an eye (E) comprising:
• a light source (LSo) positioned with respect to the eye (E) and configured to emit an illuminating light ray (r_ill) towards the eye (E);
• an optical relay (OR) positioned with respect to the eye (E) and configured to receive an incident light ray (r_inc) resulting from the interaction between the illuminating light ray( r_ill) and the eye (E), wherein
■ the incident light ray (r_inc) intersects the optical relay (OR) at an intersection point (P_int), and
■ forms an incidence angle (alpha_inc) with the normal (n_OR) to the optical relay at the point (P_int) in an incidence plane (PI_inc) formed by the incident light ray (r_inc) and the normal n_OR;

• a light sensor (LSe) positioned with respect to the optical relay (OR) and configured to receive a deflected light ray(r_def) resulting from the interaction between the incident light ray (r_inc )and the optical relay (OR), wherein
■ the deflected light ray (r_def) forms a deflection angle (alpha_def) with the normal (n_OR) in a deflection plane (PI_def) formed by the deflected light ray (r_def) and the normal (n_OR);

wherein the incidence angle (alpha_inc) is different from the deflection angle (alpha_def) and the incidence plane (PI_inc) is different from the deflection plane (PI_def); or the incidence angle (alpha_inc) is equal to the deflection angle (alpha_def) and the incidence plane (PI_inc) is different from the deflection plane (PI_def); or the incidence angle (alpha_inc) is different from the deflection angle (alpha_def) and the incidence plane (PI_inc) coincides to the deflection plane (PI_def).

## Description

The present invention relates to a system and device for an observation of an eye and its surrounding area and to the use of such system and device in applications thereof.

Systems for an observation of an eye are well known in the art. Such systems are used in a number of applications, such as eye-tracking, medical diagnosis, and drowsiness monitoring.

Drowsiness is a major cause of accidents of various types, often with grave consequences. For example, drowsiness is reported to cause 20 to 30 % of all car accidents, and, in the USA, 100,000 road accidents per year with 1,000 deaths and 71,000 injured. In addition, 6 to 11 % of the population is reported to suffer from excessive daytime sleepiness, constituting a permanent potential danger. Drowsiness while driving is clearly a major problem of public safety and security. It is thus not surprising that there is an increasing trend for authorities to launch campaigns to prevent drowsiness at the wheel, and by companies to develop drowsiness monitoring systems to equip cars and drivers.

In general, there are various approaches for the observation of an eye. In the case of drowsiness monitoring, there are two main types of systems. In the first type of systems, the system sends pulses of light towards the eye, senses the reflected light, and analyses the corresponding electrical signals. Such systems fall in a category of OptoOculoGraphy, abbreviated as OOG. In the second type of systems, the system captures images of the eye with an imaging sensor, typically a camera. Such systems fall in the category of PhotoOculoGraphy, abbreviated as POG. While OOG systems naturally illuminate the eye at each time a pulse is sent out, POG systems also tend to illuminate the eye in an active and controlled way to insure a constant illumination in all light-level conditions, including in darkness. OOG and POG systems generally use the infrared (IR) part of the electromagnetic spectrum for the simple reason that illuminating the eye in the IR does not interfere with the normal vision of the user, since the eye does not see in the IR. One should, however, be careful to respect all norms related to the IR illumination of the eye to avoid injuries to this organ.

An advantage of using POG systems and therefore images, is that this allows the monitoring system to distinguish between important parts of the eye area such as a pupil, an iris, and eyelids.

Particularly the use of cameras provides the advantage to offer both spatial resolution and temporal resolution; whereas OOG systems provides temporal resolution only.

For both OOG and POG, one can further distinguish between systems that are mounted on the head of the user and systems that are remotely placed, such as on a dashboard. The systems mounted on the user have several advantages such as providing higher spatial resolution (because of the closer distance between the eye and the camera), not requiring that the face and the eye be detected and tracked from a distance), and remaining effective even if the wearer leaves the wheel, as is conceivable in a freight train in an isolated area of a large country.

US 2008/0030685 A1 by Fergason et al. discloses a system for monitoring eye movements through an optical observation of an eye. The disclosed system comprises an optical device having a light source configured for emitting light along a first path and a sensor positioned to receive light from a second path parallel to the first path. A reflector is located within a lens and configured to reflect light emitted by the light source onto the eye and reflect light reflected by the eye to the sensor.

The problem with such a system is that the light source must necessary illuminate the eye via the reflector or reflective surface. The light source cannot illuminate the eye directly.

Moreover the system requires that the path of light sent to the eye and the path of light received from the eye must necessarily go via the reflector or reflective surface and must nearly coincide. This is unnecessarily restrictive, wasteful of energy, and may result in excessive light reaching the eye, with the danger of exposing it to excessive IR radiation.

In addition, the presence of some type of reflector, such as a hot mirror, in a lens generally results in the reflector being less transparent than the surrounding lens region and consequently visible within the field of view of the user.

Endly, a major problem that affects the system of the art, is the fact that the reflector (or reflective) surface is always - explicitly or implicitly - considered to be acting as a mirror. The terms "reflector", "reflection", and the like, imply that the relation between any light ray incident on the reflecting surface and the corresponding redirected ray is highly constrained by some basic laws of optics.

The first constraint from optics is that incident ray, reflected ray, and normal to the reflecting surface at a point of intersection of the incident ray with the reflecting surface must be in the same plane. This can be stated in an equivalent way by saying that the plane defined by the incident ray and the normal must be the same as the plane defined by the reflected ray and the normal.

The second constraint from optics is that - within the plane containing the incident ray, the normal, and the reflected ray - the angle of incidence (defined as the customary angle between the incident ray and the normal) must be equal to the angle of reflection (defined as the customary angle between the reflected ray and the normal).

These two constraints from optics severely limit the freedom of configuration in positioning the various elements used for the system for an observation of an eye. Particularly, a light source, a reflector or reflecting surface, a light sensor, and possibly a filter should be carefully positioned with respect to the eye, in a configuration that can itself be very tight and constrained, as will likely be the case if these elements must interact on a spectacles frame, which is furthermore typically articulated.

We have now found a system for an observation of an eye that overcomes the problems of the state of the art and that also provides useful properties to the reflecting surface. The system can be implemented with light pulses (OOG systems) and with images (POG systems)

In accordance with the present invention, there is provided a system for an observation of an eye (E) comprising
- a light source (LSo) positioned with respect to the eye (E) and configured to emit an illuminating light ray (r_ill) towards the eye (E);
- an optical relay (OR) positioned with respect to the eye (E) and configured to receive an incident light ray (r_inc) resulting from the interaction between the illuminating light ray (r_ill) and the eye (E), wherein
   ■ the incident light ray (r_inc) intersects the optical relay (OR) at an intersection point (P_int), and
   ■ forms an incidence angle (alpha_inc) with the normal (n_OR) to the optical relay at an intersection point (P_int) in an incidence plane (PI_inc) formed by the incident light ray (r_inc) and the normal (n_OR);
- a light sensor (LSe) positioned with respect to the optical relay (OR) and configured to receive a deflected light ray (r_def) resulting from the interaction between the incident light ray (r_inc ) and the optical relay (OR), wherein
   ■ the deflected light ray (r_def) forms a deflection angle (alpha_def) with the normal (n_OR) in a deflection plane (PI_def) formed by the deflected light ray (r_def) and the normal (n_OR);
wherein the incidence angle (alpha_inc) is different from the deflection angle (alpha_def) and the incidence plane (PI_inc) is different from the deflection plane (PI_def); or
the incidence angle (alpha_inc) is equal to the deflection angle (alpha_def) and the incidence plane (PI_inc) is different from the deflection plane (PI_def); or the incidence angle (alpha_inc) is different from the deflection angle (alpha_def) and the incidence plane (PI_inc) coincides with the deflection plane (PI_def).

By eye, one means an eyeball and its components which comprise a pupil, an iris, a sclera, a cornea, and, a retina. With the term eye, one may also include a region surrounding the eyeball, which comprises an upper eyelid, a lower eyelid, one or a plurality of skin patches and folds, and one or two eyebrows. By eye one also means any combination of an eyeball, the elements thereof and a region surrounding the eyeball.

By light source (LSo), one means one or more individual source of light or more generally, of electromagnetic radiation. The light provided by each individual source may have specific time, frequency (and thus wavelength), polarization and coherence characteristics.

Concerning the time characteristics, the light may have a modulation, such as amplitude modulation, frequency modulation, pulse-width modulation, and modulation by orthogonal codes.

Concerning the frequency characteristics or its corresponding wavelength characteristics, the light may have a spectrum that consists of one or more frequencies or of frequency bands at one or more frequencies, with frequency bandwidths that may be relatively narrow. Preferably, the wavelength is chosen in the infrared part of the electromagnetic spectrum, just outside the visible wavelengths. More preferably, the wavelength of the light source is in the range of 800 nm and 3000 nm. Most preferably, the wavelength of the light source is 870nm.

Concerning the polarization characteristics, the light may be unpolarized, partially polarized, or polarized. The polarization may be linear or circular. Preferably, the light source is un-polarized.

Concerning the coherence, the light may be incoherent, partially coherent, or coherent, in time and/or space. Preferably, the light is incoherent.

When using several sources of light, each source of light may have the same or different time, frequency, polarization, and coherence characteristics. They may emit at the same time or at different times. Preferably, each light source comprises a light emitting diode (LED).

The invention is described in terms of light rays or, simply, rays, such as an illuminating light ray (r_il), an incident light ray (r_inc), and a deflected light ray (r_ref). But "ray" also means "beam". It should be clear that the description in terms of rays is a significant simplification of the actual propagation of electromagnetic waves, governed by Maxwell's equations and by the corresponding equations of wave propagation. While the conceptual and practical notion of ray is used in geometrical optics, more precise approximations of Maxwell's equations are used, e.g., in Fourier optics and in physical optics. The use of rays has the advantage to permit a simple, concise description of the invention.

More precisely, by deflected light ray (r_def), one means a light ray that is deflected by an optical relay in a way that is not constrained by the basics laws of optics, as explained above. The terms "to deflect", "deflected", and the like comprise a meaning of changing a direction of the light ray when the light ray hits a surface.

By incidence angle (alpha_inc), one means the angle formed by the incident ray with the normal to a surface of the optical relay at the point of intersection of the incident ray with the surface of the optical relay.

By deflection angle (alpha_def), one means the angle formed by the deflected ray with the normal to a surface of the optical relay at the point of intersection of the deflected ray with the surface of the optical relay.

By light sensor (LSe), one means a device for sensing light, a device for collecting photons, a device for making images, an imaging sensor, an imaging device, or a camera, which all produce data related to an eye, comprising signals, images, images-like data, image sequences, and videos. When the light sensor is used to produce images or image-like data of an eye, the light sensor may comprise a sensing surface which has some depth and is typically planar, and optical elements configured to bring each deflected ray (r_def) to the appropriate location on the sensing surface. Each deflected ray may contribute to the intensity level at one or a plurality of image elements, called pixels. Particularly, the light sensor collects deflected rays (r_def) or images from eyelids, pupils, iris and the like.

By optical relay (OR), one means a device made of a surface or a volume having one or more of the following capabilities, which may be wavelength dependent: a deflection capability, a transmission capability, an absorption capability, a transparency capability, a refraction capability, a diffraction capability, a focusing capability, an imaging capability, and a selectivity capability. These capabilities are convenient ways of referring to specific transformations of one or a plurality of incident light rays governed by Maxwell's equations. Some of the above capabilities are related to each other.

Preferably, the optical relay (OR) according to the invention has a wavelength-dependent diffraction capability.

By deflection capability, one means the capacity of the optical relay to change the light ray direction in a way that does not obey the basic laws of reflection optics with respect to the surface of the optical relay.

By transmission capability, one means the capacity of the optical relay to allow the incident light to traverse its surface, generally with some attenuation.

By absorption capability, one means the capacity of the optical relay to absorb some of the incident light. Preferably, the absorption capability should not exceed 20% in the visible wavelength.

By transparency capability, one means the capability of the optical relay to allow most of the incident light to traverse the optical relay at a specific wavelength. Preferably, the optical relay is transparent at visible wavelengths.

By refraction capability, one means the capability of the optical relay to change the direction of incident light at one or a plurality of interfaces between adjacent materials having different indices of refraction.

By diffraction capability, one means the capability achieved by the optical relay by using diffraction gratings such as a holographic grating. The diffraction capability may enable other capacities such as deflection capability, focusing capability, imaging capability and selection capability.

By focusing capability, one means the capacity of the optical relay to make a set of parallel, or nearly parallel, incident light rays to focus at a common point, of the light sensor (Lso).

By imaging capability, one means the capacity of the optical relay to form an image at the output of the light sensor (Lso) from a set of incident light rays.

By selectivity capacity, one means the capacity of the optical relay to perform some operations, such as deflection, at a specific frequency or band of frequencies. Preferably, the selectivity capacity of the optical relay refers to deflection, focusing, and/or imaging in the IR, while being simultaneously transparent in the visible.

More practically, the optical relay may be a molded insert.

The optical relay may also be applied to one surface of a lens in such a way that it is removable.

Alternatively, the optical relay may be imprinted to a surface of a lens or in the volume of a lens.

The optical relay may comprise one or several optical coatings.

Generally, the optical relay is a holographic element or, synonymously, a diffractive element.

The holographic element may be a surface hologram or a volume hologram. The holographic element may be implemented using silver-halide material and techniques or dichromated-gelatin (DCG) material and techniques as described by T G. Georgekutty and H. Liu, in Appl. Opt. 26, 372-376 (1987).

Holographic elements (HEs) can be created on the surface of the optical relay (surface hologram) or within the volume (generally near the surface) of the optical relay (volume hologram). The holographic effect is obtained by creating diffractions patterns or arrays on the surface or in the volume, as applicable. These arrays are often called Bragg arrays. Each array has a particular spatial periodicity, (and, thus, corresponding spatial period and spatial frequency) which makes it to exhibit its special behavior, such as focusing, at a specific temporal frequency corresponding to this spatial period. The period of the array must therefore be adapted to the frequency (and, thus, wavelength) of interest, such as a particular IR frequency. HEs exhibit their special behavior in a fairly narrow range of frequencies. By using a small number of distinct periods, one can make the HE to exhibit its special behavior at the same number of distinct frequencies or frequency bands.

In the case of volume holograms, which is our preferred way of implementing HEs in the context of the present invention, one often talks about Bragg planes (or surfaces).

The fact that an HE exhibits its special behavior at (and near) a specific frequency is particularly useful. For example, this allows an HE to work as a deflector or imager at a specific frequency, such as at an IR frequency, and to be essentially transparent at all other frequencies, in particular in the visible. This is exactly what is relied upon when illuminating the eye in the IR and recording the corresponding IR images of the eye via an HE placed in the field of vision of the user.

Particularly, an HE performs deflection and focusing capabilities, on the IR light coming from an eye as a result of it being illuminating in the IR by the light source, while being simultaneously transparent or quasi transparent in the visible, which allows a user to see through the HE placed in his field of vision, and the HE to be invisible to the user.

One advantage of the present invention is that the system is not limited by both optical constraints of the state of the art. The system allows a greater freedom of configuration between the light source, the optical relay, and the light sensor.

The light source, the light sensor, and the optical relay according to the invention are configured to perform an optical observation of one eye; but the system may be duplicated to allow for the optical observation of each of both eyes. The system of an observation of an eye according to the invention may also be extended to the observation of other parts of the user such as head, parts thereof, or other body elements.

In a preferred embodiment, the light source, the optical relay, and the light sensor of the system for an observation of an eye according to the invention are connected to a support. The support may be fixed or mobile.

The present invention also concerns a device for an observation of an eye (E) comprising:
- a frame (FR) configured to be worn on a user;
- a light source (LSo) connected to the frame and positioned with respect to the eye (E) to emit an illuminating light ray (r_ill) towards the eye (E);
- an optical relay (OR) connected to the frame and positioned with respect to the eye (E) to receive an incident light ray (r_inc) resulting from the interaction between the illuminating light ray( r_ill) and the eye (E), wherein
   ■ the incident light ray (r_inc) intersects the optical relay (OR) at an intersection point (P_int), and
   ■ forms an incidence angle (alpha_inc) with the normal (n_OR) to the optical relay at an intersection point (P_int) in an incidence plane (PI_inc) formed by the incident light ray (r_inc) and the normal n_OR;
- a light sensor (LSe) connected to the frame and positioned with respect to the optical relay (OR) to receive a deflected light ray(r_def) resulting from the interaction between the incident light ray (r_inc )and the optical relay (OR), wherein
   ■ the deflected light ray (r_def) forms a deflection angle (alpha_def) with the normal (n_OR) in a deflection plane (PI_def) formed by the deflected light ray (r_def) and the normal (n_OR);
wherein:
the incidence angle (alpha_inc) is different from the deflection angle (alpha_def) and the incidence plane (PI_inc) is different from the deflection plane (PI_def); or
the incidence angle (alpha_inc) is equal to the deflection angle (alpha_def) and the incidence plane (PI_inc) is different from the deflection plane (PI_def); or
the incidence angle (alpha_inc) is different from the deflection angle (alpha_def) and the incidence plane (PI_inc) coincides with the deflection plane (PI_def).

In a preferred embodiment, the light source, the optical relay, and the light sensor of the device for an observation of an eye according to the invention are connected to a frame to be worn by a user on his head.

More particularly, the device can be configured in such a way that the frame and the elements connected to it can be used over conventional prescription glasses or sunglasses.

The frame of the device according to the invention may comprise a front piece and at least one side piece such as in the frame of glasses, spectacles, eye glasses, goggles, or equivalent. The front piece may comprise a support and one or two lenses or glasses made of glass or plastic

On such a frame, the light source is preferably arranged on the front piece and directed towards an eye of the user. The light source is most preferably arranged at the bottom of the front piece of the frame, and, particularly, out of the field of vision of the person.

On such a frame, the optical relay is preferably arranged on the front piece of the frame and most preferably integrated in the lens or glass located in front of the observed eye if the transparency capability of the optical relay is high enough. Most preferably, the optical relay is integrated in the eye glass in front, or nearly in front, of the observed eye.

On such a frame, the light sensor (Lso) is preferably positioned on the sidepiece of the frame closer to the observed eye in such a way as to receive as much as possible of the light coming from the eye as a result of its illumination by the light source. Since the light source, optical relay, and light sensor are preferably configured to operate at a specific frequency, preferably in the IR, and to some degree in a narrow frequency band around the specific IR frequency; the light sensor will record essentially the deflected IR light resulting from the interaction between the illuminating IR light ray and the eye.

A filter for removing light in the visible spectrum may be added in front of the sensor.

The system and device according to the invention may also comprise processing means for converting an output from the light sensor into analog or digital data information about the state of the eye of the user.

It may further be used to obtain data about the state of the body or mind of the user.

The system and device may further comprise an ambient-light sensor producing an output signal that can be used to control one or more of the light sources and the output of the light sensor.

The system and device may comprise an ambient-light sensor producing an output signal that can be used by the processing means, for example to control one or more of the light sources, the output of the light sensor, and the operation of the processing means.

The device according to the invention may further comprise an attitude sensor or a plurality of attitude sensors that provide the position and orientation of the frame with respect to the physical world.

The device may also comprise another sensor that provides information about the environment, such as a camera providing images of the environment in front of the user.

Finally, the present invention also refers to all applications using the system or device for an observation of an eye such as system or device for monitoring of drowsiness, alertness, fatigue, somnolence, alertness, wakefulness, distraction, inattention, or equivalent of the user.

The system or device may also be used in applications using eye-tracking or the knowledge of which direction the user is looking into with respect to the frame or with respect to the physical environment or both, such as in psychological studies, in market research, or for the remote selection of items. The system or device may further be used in applications requiring images of the interior of the eye, for example of the retina, such as in ophthalmology.

The invention will now be illustrated in detail with reference to the drawings. The following description is presented to enable one of ordinary skill in the art to make and use the invention. Descriptions of specific embodiments and applications are provided only as examples. But the present invention is not limited to such embodiments and applications. Other embodiment and applications are possible without departing from the scope of the invention

A very accurate numerical simulation of the invention is performed in a computer by using advanced ray-tracing software packages such as ASAP provided by Breach Research. The software has been used to illustrate the invention hereafter.

### Brief description of the drawings:

Fig 1 shows a schematic drawing of the system according to the invention.
Fig 2 to 4 show schematic drawings of the optical relay according with three different configurations according to the invention
FIG 5 and Fig 6 show schematic drawings illustrating the focusing capability of an optical relay (OR) according to the invention.
Fig 7 shows a schematic drawing illustrating the imaging capability of an optical relay (OR) according to the invention.
Fig 8 shows a schematic drawing of one device according to the invention.
Fig 9 shows a practical implementation according to the invention.

In the description of the drawings, the following elements are used with their related definition:
- a light source (LSo), comprising at least one individual source of light, preferably in the infrared (IR) part of the electromagnetic spectrum,
- an eye (E),
- an optical relay (OR) with at least the basic capability of deflecting an incident (light) ray (r_inc) into a specified direction, by exploiting either its surface properties, or its volume properties, or both,
- a deflecting surface (DS), which is typically, but without limitation, a reference plane corresponding to, and/or parallel to, an outside surface of the optical relay (OR),
- a light sensor (LSe),
- an illuminating (light) ray (r_ill),
- an incident (light) ray (r_inc) which is the incident light ray to the_ optical relay (OR) and which corresponds to the illuminating ray (r_ill),
- a deflected (light) ray (r_def) which is the deflected light ray from the optical relay (OR) and corresponding to the incident ray (r_inc),
- an intersection point (P_int), defined as the intersetion of the incident ray (r_inc) and the optical relay (OR) and/or the deflecting surface (DS),
- a normal (i.e. a perpendicular) (n_OR) to the optical relay and/or the deflecting surface (DS) at the intersection point (P_int),
- an incidence plane (PI_inc), defined by the normal (n_OR) and the incident ray (r_inc),
- a deflection plane (PI_def), defined by the normal (n_OR) and the deflected ray (r_def),
- an incidence angle (alpha_inc), defined as the customary angle between the normal (n_OR) and the incident ray (r_inc),
- a deflection angle (alpha_def), defined as the customary angle between the normal (n_OR) and the deflected ray (r_def),
- an incidence trace (Tr_inc), defined as the line of intersection of the incidence plane (PI_inc) and the deflecting surface (DS),
- a deflection trace (Tr_def), defined as the line of intersection of the deflection plane (PI_def) and the deflecting surface (DS),
- a reference x-axis (x),
- a reference y-axis (y), orthogonal to the x-axis (x) and forming a right-handed system with the x-axis (x),
- an angle (phi_inc), defined as the customary angle between the incidence trace (Tr_inc) and the x-axis (x),
- an angle (phi_def), defined as the customary angle between the deflection trace (Tr_def) and the x-axis (x),
- a difference angle (Delta_phi), defined as the difference between the angle phi_def and the angle phi_inc,
- a bundle of rays, or ray bundle (BR), defined as being a set of rays all oriented in the same direction,
- a focal point (FP), defined as the point through which all the rays in a ray bundle (BR) incident on an optical relay (OR) go through after deflection by the optical relay (OR),
- a frame (FR), i.e. a support,
- a lens (LE), as found, e.g., in a pair of eye glasses.

### Detailed description of the drawings:

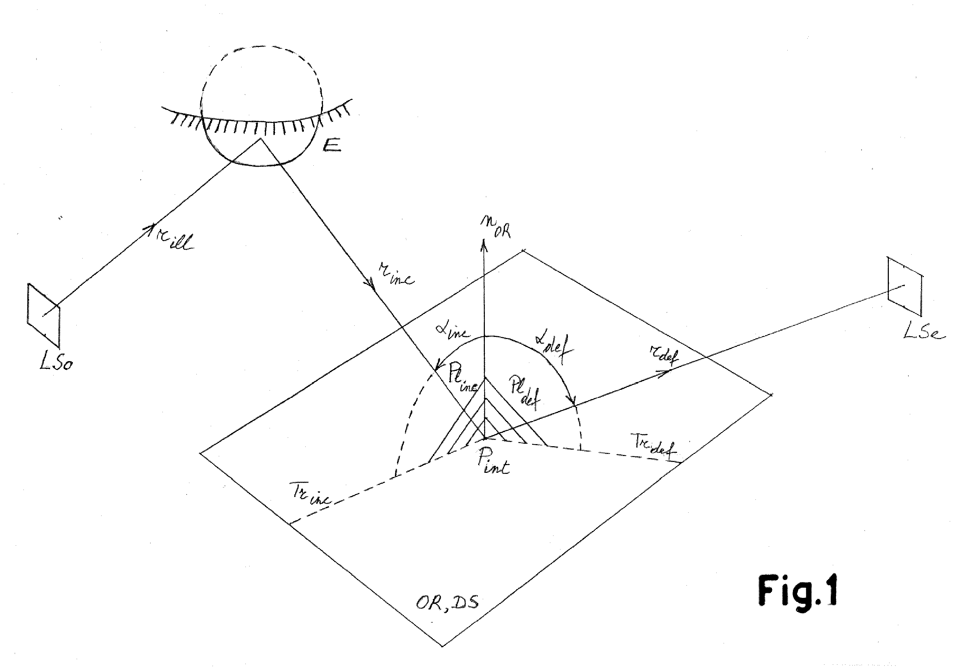

Figure 1 shows a schematic drawing of a system according to the invention. The figure shows a light source (LSo), an eye (E), an optical relay (OR), and a light sensor (LSe). The figure also shows a deflecting surface (DS), which is typically a reference plane corresponding to, and/or parallel to, an outside surface of the optical relay (OR). An illuminating ray (r_ill) is produced by the light source (LSo) and further illuminates an eye (E). After interaction with the eye (E), this ray gives rise to an incident ray (r_inc) that, intersects the deflecting surface (DS) at an intersection point (P_int). After interaction with the optical relay (OR), this ray gives rise to a deflected ray (r_def) that reaches the light sensor (LSe). The normal (n_OR) to the deflecting surface (DS) at the intersection point (P_int) and the incident ray (r_inc) define an incidence angle (alpha_inc) and an incident plane (PI_inc). The same normal with the deflected ray (r_def) define a deflection angle (alpha_def) and a deflection plane (PI_def). According to the invention, the incidence angle (alpha_inc) differs from the deflection angle (alpha_def) and/or the incidence plane (PI_inc) differs from the deflection plane (PI_def). Since the incidence plane (PI_inc) contains the normal (n_OR) to the deflecting surface (DS), it is perpendicular to the deflecting surface (DS). The same is true for the deflection plane (PI_def). The intersection of the incidence plane (PI_inc) and the deflecting surface (DS) is referred to as the incidence trace (Tr_inc). The intersection of the deflection plane (PI_def) and the deflecting surface (DS) is referred to as the deflection trace (Tr_def).

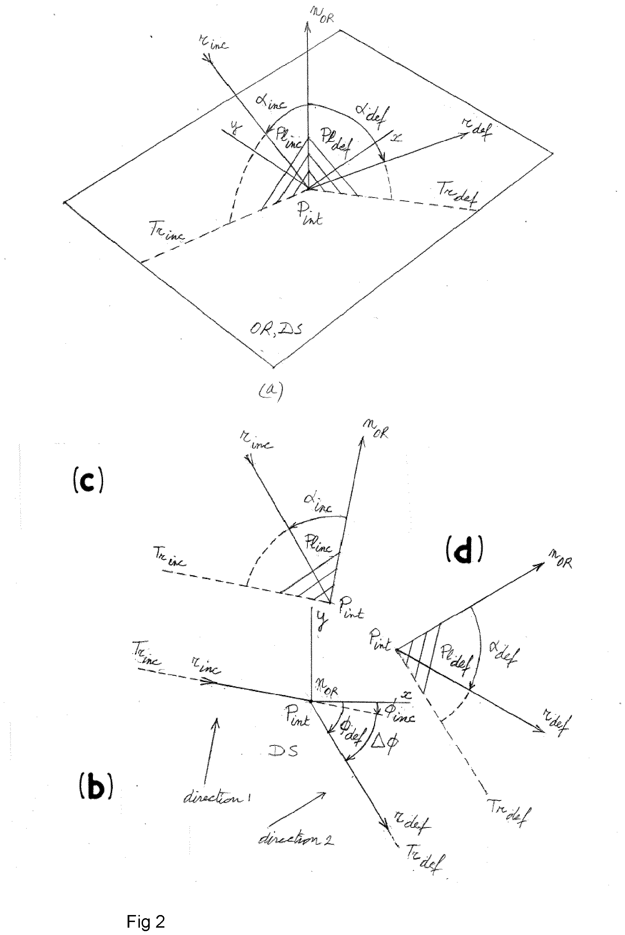

Figure 2(a) reproduces a first embodiment of the system according to the invention wherein the incidence plane (PI_inc) differs from the deflection plane (PI_def) and the incidence angle (alpha_inc) differs from the deflection angle (alpha_def).
   Reference axes are introduced in Figures 2(a), for defining the orientation of planes perpendicular to the deflecting surface (DS). These axes are referred to as x-axis (x) and y-axis (y), and are orthogonal and right-handed,
Figure 2(b) shows a top view - also called a plan view - of Figure 2(a), highlighting two specific viewing directions. Viewing direction 1 relates to r_inc whereas viewing direction 2 relates to r_def.
Figures 2(c) and 2(d), are elevation views - also called front views- of respectively direction 1 and direction 2.
Figure 2(b) shows an angle (phi_inc) between the x-axis (x) and the incidence trace (Tr_inc), an angle (phi_def) between the x-axis (x) and the deflection trace (Tr_inc), and a difference angle (Delta-phi) between these last two angles, i.e. Delta_phi = phi_def - phi_def. These angles are signed quantities defined in a customary way. The difference angle (Delta_phi) is thus the angle between the incidence plane (PI_inc) and the deflected plane (PI_def). As Figure 2 corresponds to the case where the incidence plane (PI_inc) differs from the deflected plane (PI_def), the difference angle (Delta_phi) differs from zero, whether this angle is expressed in degrees, radians, or some other units.
   In Figure 2(c), the viewing direction 1 of Figure (2b) is reproduced. It is perpendicular to the incidence trace (Tr_inc). The incidence angle (alpha_inc) is 40 degrees.
   In Figure 2(d), the viewing direction 2 of Figure (2b) is reproduced. It is perpendicular to the deflected trace (Tr_def). The deflection angle (alpha_def) is 60 degrees.

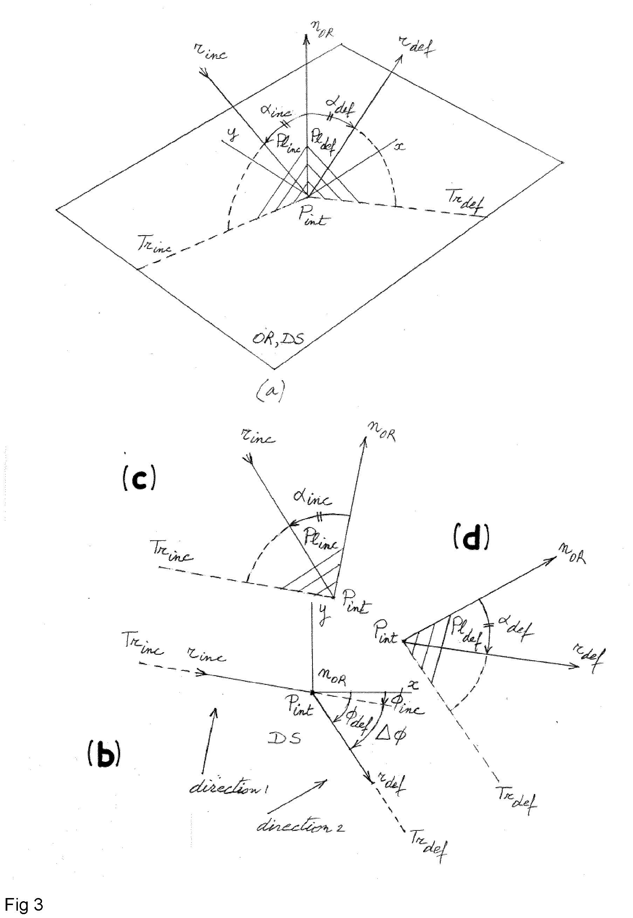

Figure 3(a) reproduces a second embodiment of the system according to the invention wherein the incidence plane (PI_inc) differs from the deflection plane (PI_def) and the incidence angle (alpha_inc) is equal to the deflection angle (alpha_inc). In such embodiment, the difference angle (Delta-alpha) is zero.
Figure 3(b) shows a top view - also called a plan view - of Figure 3(a), highligting two specific viewing directions. Viewing direction 1 relates to r_inc whereas viewing direction 2 relates to r_def.
Figures 3(c) and 3(d), are elevation views - also called front views- of direction 1 and direction 2 as already defined in Figs 2 but with angle (alpha_inc) equal to the deflection angle (alpha_inc).

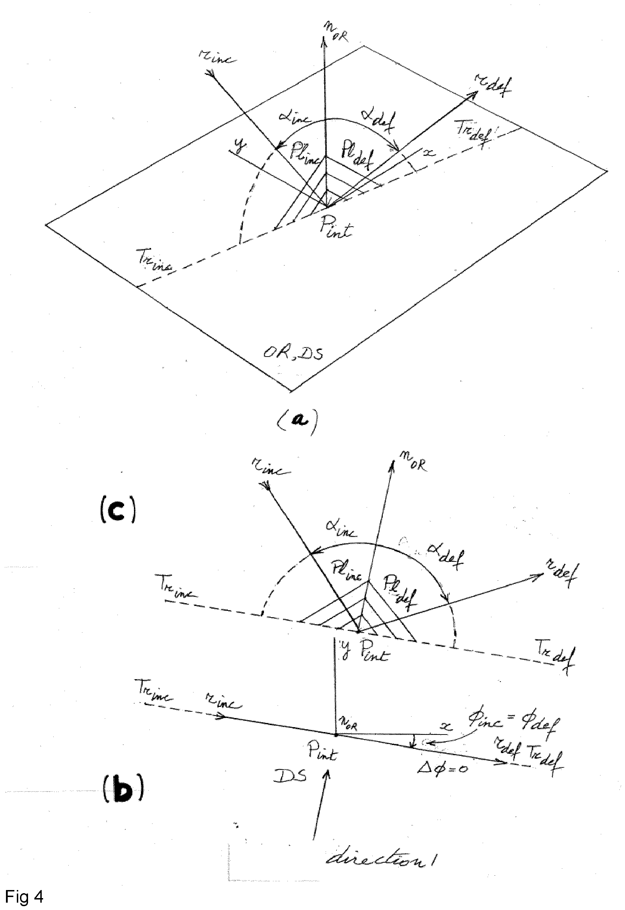

Figure 4(a) reproduces a third embodiment of the system according to the invention wherein the incidence plane (PI_inc) coincides with the deflection plane (PI_def) and the incidence angle (alpha_inc) differs from the deflection angle (alpha_inc). In the present case, the difference angle (Delta_alpha) differs from zero, but (Delta_phi) equal zero.
Figure 4(b) shows a top view - also called a plan view - of Figure 4(a), highligting one viewing direction. Viewing direction 1 relates to r_inc and to r_def because both incidence plane (PI_inc) and the deflection plane (PI_def) coincide.
Figures 4(c), is an elevation view - also called front view- of direction 1 wherein the incidence angle (alpha_inc) differs from the deflection angle (alpha_inc).

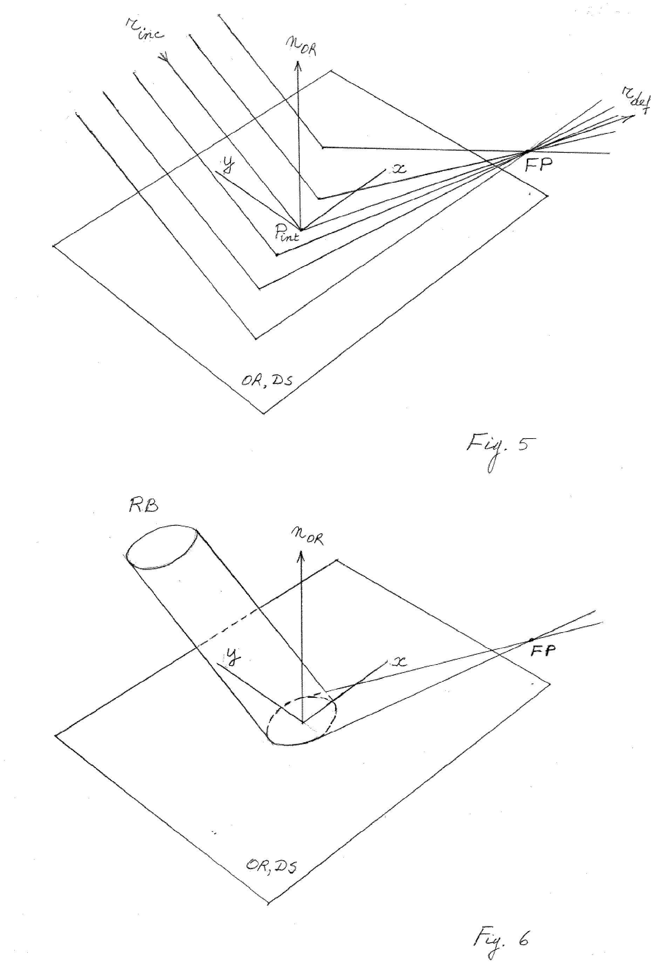

Figure 5 illustrates the focusing capability of an optical relay (OR). The figure shows that each incident ray (r_inc) in a ray bundle (RB) incident on the optical relay (OR) is deflected in such a way that all the rays in the ray bundle (RB) go through a common point, called a focal point (FP).

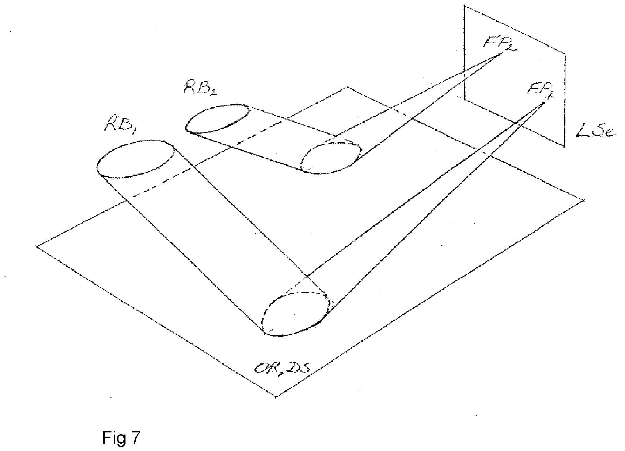

Figure 6 also illustrates the focusing capability of an optical relay (OR), but in a more concise way than in Figure 5. The parallel incident rays (r_inc) in the ray bundle (RB) are indeed represented as a cylinder, and the corresponding set of deflected rays as a cone This concise representation is used advantageously in Figure 7.
Figure 7 illustrates the imaging capability of an optical relay (OR). The figure indeed shows that the optical relay (OR) focuses ray bundles (here, RB_1 and RB_2) corresponding to different travel directions on generally different focal points (here, FP_1 and FP_2), all located on the light sensor (LSe).

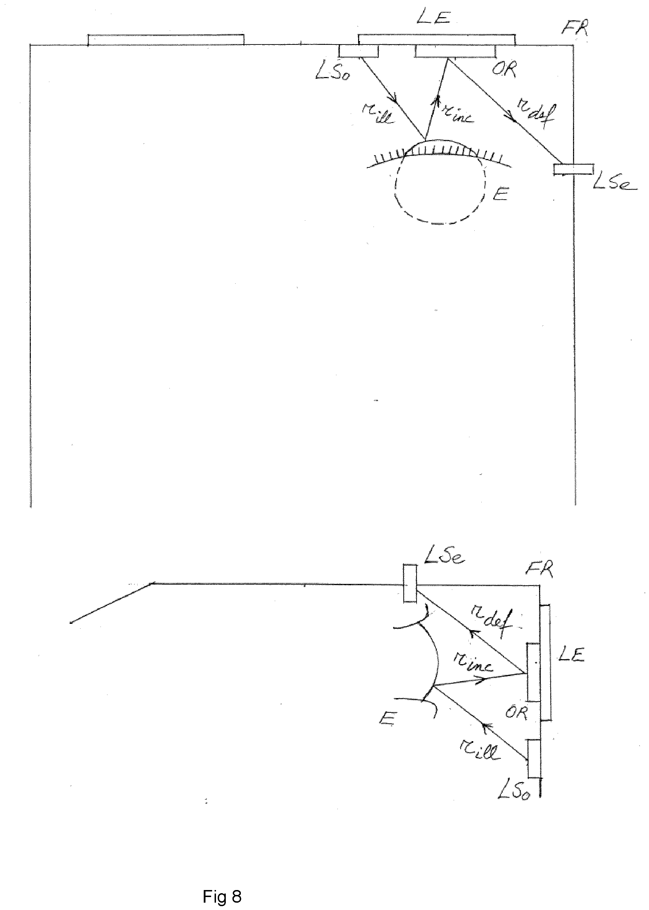

Figure 8 shows a schematic drawing of a device according to the invention. The figure shows a frame (FR) configured to be worn by a user, a light source (LSo) connected to the frame (FR) and positioned to emit an illuminating ray (r_ill) towards eye (E).

An optical relay (OR) is also connected to the frame and is positioned with respect to the eye to receive an incident ray(r_inc) resulting from the interaction of (r_ill) with the eye (E). Finally a light sensor (LSe) is also connected to the frame (FR) to receive a deflected light ray (r_def). All three elements are attached directly or indirectly to a frame (FR). If the frame is part of a pair of eyeglasses, the optical relay (OR) is preferably mounted on one of the lenses (LE) of the eyeglasses.

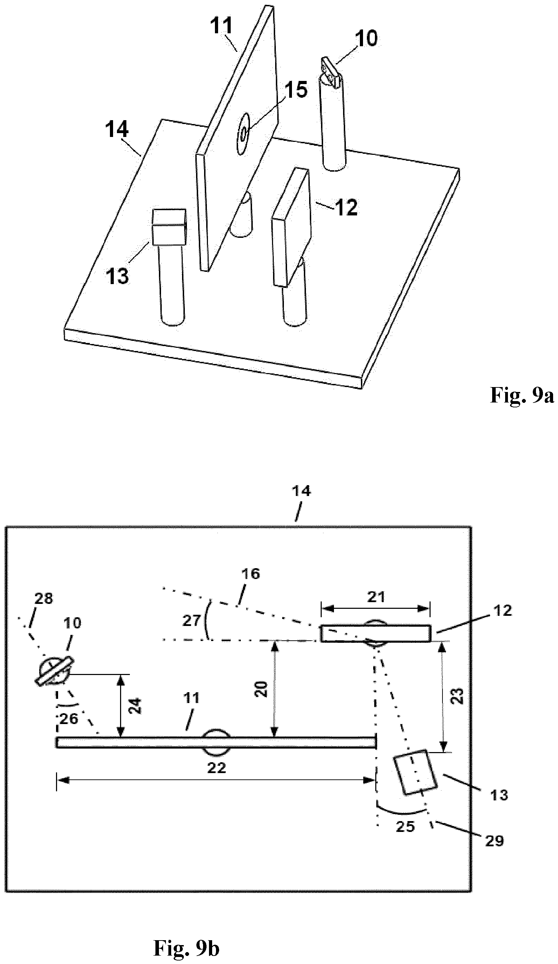

Figure 9 illustrates a practical implementation of the invention.

Two infrared emitters (10) provided by Vishay Semiconductors with a commercial reference: VSMF4720 and having a Peak wavelength at 870 nm with a Spectral bandwidth of 40 nm have been positioned on an optical table (14) to illuminate a picture of an eye (11) of dimension 88 mm (22) x 52 mm.

A video camera (13) provided by Supercircuits with the commercial reference PC206XP and having an Image sensor type: B/W Cmos was also attached to the optical table (14), to film a diffractive element (12) of dimension 30 mm (21) x 30 mm. The camera is equipped with a filter provided by Präzisions Glas & Optik with commercial reference SCHOTT RG 830 and having 50% of transmittance at 830 nm.

The diffractive element (12) is an holographic grating with a Peak wavelength of diffracted spectrum at 850 nm and an angle (β) of 20° (27) between the grating direction (16) and the holographic surface (here the top surface or any parallele surface to the top surface) as shown hereafter. 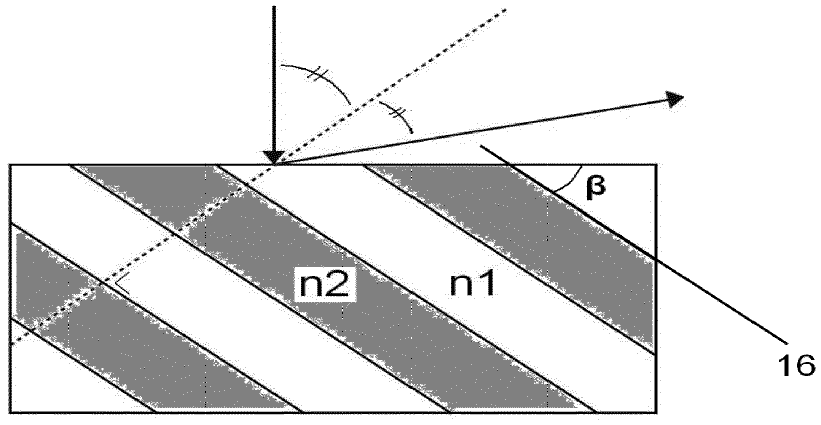 diffractive element (12)

Both infrared emitters (10) are placed at 20 mm (24) of the eye (11 ) with an angle of 40° (26) between an emission axis (28) of both infrared emitters (10) and the perpendicular to the surface of (11). The diffractive element (12) is placed at 30 mm (20) of the eye (11) and 35 mm (23) of the camera (13). The angle between the viewing axis (29) of the camera (13) and the perpendicular to the surface of the diffractive element (12) is 10° (25). The grating direction (16), the emission axis (28), the viewing axis (29) of the camera (13) and the center (15) of the diffractive element (12) are in the same plan. This plan runs parallel to the surface of (14).

The selectivity of the holographic grating: diffraction efficiency vs. wavelength(nm) is reported hereafter: 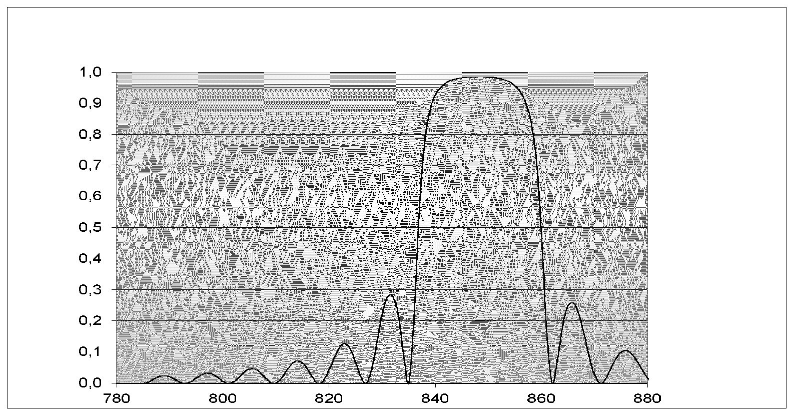 and the selectivity of the holographic grating : diffraction efficiency vs. angle of incidence (degree) is reported hereafter

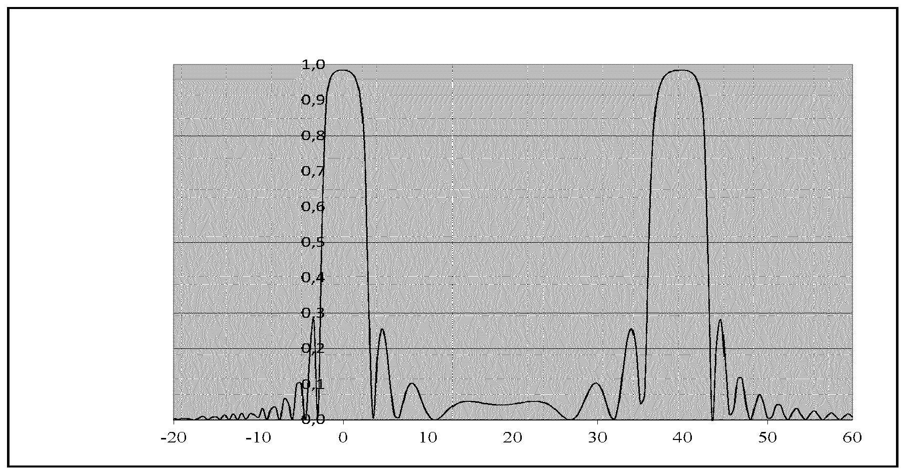

The holographic grating exhibit a maximum efficiency at 0° and 40° incidence, symmetrically with respect to the normal to the reference surface (β) Consequently, in the above configuration, the angle of incidence is zero degree and the deflection angle is 40 degree.

## Claims

1. A system for an observation of an eye (E) comprising:
• a light source (LSo) positioned with respect to the eye (E) and configured to emit an illuminating light ray (r_ill) towards the eye (E);
• an optical relay (OR) positioned with respect to the eye (E) and configured to receive an incident light ray (r_inc) resulting from the interaction between the illuminating light ray( r_ill) and the eye (E), wherein
■ the incident light ray (r_inc) intersects the optical relay (OR) at an intersection point (P_int), and
■ forms an incidence angle (alpha_inc) with the normal (n_OR) to the optical relay at the point (P_int) in an incidence plane (PI_inc) formed by the incident light ray (r_inc) and the normal n_OR;
• a light sensor (LSe) positioned with respect to the optical relay (OR) and configured to receive a deflected light ray(r_def) resulting from the interaction between the incident light ray (r_inc )and the optical relay (OR), wherein
■ the deflected light ray (r_def) forms a deflection angle (alpha_def) with the normal (n_OR) in a deflection plane (PI_def) formed by the deflected light ray (r_def) and the normal (n_OR);
**characterized in that**:
the incidence angle (alpha_inc) is different from the deflection angle (alpha_def) and the incidence plane (PI_inc) is different from the deflection plane (PI_def); or
the incidence angle (alpha_inc) is equal to the deflection angle (alpha_def) and the incidence plane (PI_inc) is different from the deflection plane (PI def); or the incidence angle (alpha_inc) is different from the deflection angle (alpha_def) and the incidence plane (PI_inc) coincides to the deflection plane (PI_def).

2. The system according to claim 1 **characterized in that** the light source (LSo), the optical relay (OR) and the light sensor (LSe) are positioned to be mounted on a support.

3. The system according to claim 1 or 2 further comprising processing means for converting an output signal of the light sensor into an analog or digital information data.

4. The system according to any one of claim 1 to 3 **characterized in that** the optical relay is a holographic element, preferably a volume holographic element.

5. The system according to any one of claim 1 to 4 wherein the light source emits an illuminating light ray (r_ill) with a wavelength in the range of 800 nm to 3000 nm, preferably 870 nm.

6. The system according to any one of claim 1 to 5 further comprising a filter before the light sensor to filter visible wavelengths.

7. A device for an observation of an eye and its surrounding area comprising :
• a frame configured to be worn on a user;
• a light source (LSo) connected to the frame and positioned with respect to the eye (E) to emit an illuminating light ray (r_ill) towards the eye (E);
• an optical relay (OR) connected to the frame and positioned with respect to the eye (E) to receive an incident light ray (r_inc) resulting from the interaction between the illuminating light ray( r_ill) and the eye (E), wherein
■ the incident light ray (r_inc) intersects the optical relay (OR) at an intersection point (P_int), and
■ forms an incidence angle (alpha_inc) with the normal (n_OR) to the optical relay at point (P_int) in an incidence plane (PI_inc) formed by the incident light ray (r_inc) and the normal n_OR;
• a light sensor (LSe) connected to the frame and positioned with respect to the optical relay (OR) to receive a deflected light ray(r_def) resulting from the interaction between the incident light ray (r_inc )and the optical relay (OR), wherein
■ the deflected light ray (r_def) forms a deflection angle (alpha_def) with the normal (n_OR) in a deflection plane (PI_def) formed by the deflected light ray (r_def) and the normal (n_OR);
**characterized in that**:
the incidence angle (alpha_inc) is different from the deflection angle (alpha-def) and the incidence plane (PI_inc) is different from the deflection plane (PI_def); or
the incidence angle (alpha_inc) is equal to the deflection angle (alpha_def) and
the incidence plane (PI_inc) is different from the deflection plane (PI_def); or
the incidence angle (alpha_inc) is different from the deflection angle (alpha_def) and the incidence plane (PI_inc) coincides to the deflection plane (PI_def).

8. The device according to claim 7 further comprising processing means for converting an output signal of the light sensor into an analog or digital information data.

9. The device according to claim 7 or 8 **characterized in that** the optical relay is a holographic element, preferably a volume holographic element.

10. The device according to claim 7 to 9 wherein the light source emits an illuminating light ray (r_ill) with a wavelength in the range of 800 nm to 3000 nm, preferably 870 nm.

11. The device according to any one of claim 7 to 10 further comprising a filter positioned before the light sensor to filter visible wavelengths.

12. The device according to anyone of claims 7 to 11 further comprising an attitude sensor that provides a position and orientation of the frame with respect to the physical world.

13. The device according to anyone of claim 7 to 12 further comprising a sensor that provides information about an environment in front of the user.

14. Use of the system according to claim any one of claim 1 to 6 for a monitoring of drowsiness.

15. Use of the device according to claim any one of claim 7 to 14 for a monitoring of drowsiness.
